# EUROPEAN PATENT SPECIFICATION

(11) **EP 3 139 849 B1**
(45) Date of publication and mention of the grant of the patent: **30.09.2020**
(21) Application number: 15789941.0
(22) Date of filing: 07.05.2015
(51) Int. Cl.: A61B 17/72, A61B 17/74

(54) **HIP FIXATION WITH LOAD-CONTROLLED DYNAMIZATION**
HÜFTFIXATION MIT LASTGESTEUERTER DYNAMISIERUNG
FIXATION DE HANCHE AYANT UNE DYNAMISATION CONTRÔLÉE PAR CHARGE

(30) Priority: 07.05.2014 US 201461989662 P
(43) Date of publication of application: 15.03.2017
(60) Divisional application: 20192511.2
(73) Proprietor: Acumed LLC, Hillsboro, OR 97124 (US)
(72) Inventor: EHMKE, Larry, W., Portland, OR 97225 (US)
(74) Representative: HGF
(86) International application number: PCT/US2015/029789
(87) International publication number: WO 2015/171959

(56) References cited:
- WO-A1-2013/075730
- WO-A2-2008/098728
- WO-A2-2008/098728
- JP-B2- 4 017 211
- US-A- 6 102 951
- US-A1- 2002 173 792
- US-A1- 2006 095 039
- US-A1- 2008 177 291
- US-A1- 2008 269 752
- US-A1- 2008 281 326
- US-A1- 2012 109 128
- US-A1- 2014 058 392
- US-B1- 8 157 803

## Description

### Introduction

The hip joint is a synovial joint formed by articulation of the head of the femur (the femoral head) with the acetabulum of the pelvis. The hip joint(s) supports the weight of the body when a person is standing, walking, or running, among others.

Trauma to the femur can fracture the femur near the hip joint. Depending on the position and severity of fracture, the femoral head may be replaced with a prosthesis, or the femur may be stabilized with an implanted fixation system to stabilize the femoral head while the femur heals. The fixation system may include a plate or a nail, among others.

Components of the fixation system may be freely slidable or fixed rigidly relative to one another, to respectively allow or prevent intra-femoral movement at the fracture site. However, neither approach may be optimal. An improved hip fixation system is needed.

WO 2008/098728 A2 describes a hip fracture device having a bone plate having a plurality of openings for receiving a compression bone screw or a cortical screw. An end cap is threadably insertable in the opening and a layer of polymeric material is interposed between the end cap and the top of the head such that compression of the polymeric material allows alight axial movement of the screw.

### Summary

The present disclosure provides a system for hip fixation with load-controlled dynamization.

According to a first aspect of the present invention, there is provide a system for hip fixation as described in claim 1.

In exemplary embodiments, the system comprises a fixation element, such as a screw, configured to be placed into a proximal femur of a subject, with a leading end of the fixation element anchored in a head of the proximal femur. The system also comprises a stop member configured to be connected to the lateral portion of the proximal femur. The system further comprises a deformable member configured to be plastically deformed by compressive force exerted on at least a portion of the deformable member by the fixation element and the stop member in response to a load applied to the proximal femur by the subject, such that the fixation element and the stop member move relative to one another parallel to a long axis of the fixation element.

### Brief Description of the Drawings

Figure 1 is a front, partially sectional view of selected aspects of an exemplary hip fixation system installed in a fractured femur of a subject and configured for load-controlled dynamization through deformation of a deformable region of the system, in accordance with aspects of the present disclosure.
Figure 2 is a pair of partially sectional, fragmentary views of the fixation system of Figure 1, taken generally around the region indicated at "2" in Figure 1 and showing movement of components of the fixation system relative to one another, and associated movement of portions of the femur relative to one another, in response to a deforming load applied to the fixation system by the subject (compare panels A and B), to bring edges of a fracture into contact with one another.
Figure 3 is a view of an exemplary deformable spacer element to provide a deformable region for any of the hip fixation systems of the present disclosure, with the element having a cellular structure formed by 3-D printing to render the element crushable by plastic deformation to reduce the volume occupied by the element, in accordance with aspects of the present disclosure.
Figure 4 is a view of another exemplary deformable spacer element to provide a deformable region for any of the hip fixation systems of the present disclosure, with the element being formed by a foam, in accordance with aspects of the present disclosure.
Figure 5 is a view of still another exemplary deformable spacer element to provide a deformable region for any of the hip fixation systems of the present disclosure, with the element being formed by a polymer, in accordance with aspects of the present disclosure.
Figure 6 is a partially sectional view of an example of a hip fixation system with load-controlled dynamization, not forming part of the claimed invention, with the system attached to a fractured femur and including a support member structured as a nail and also including a fixation element extending through the nail, and with the fixation element having a deformable region configured for plastic deformation by deforming contact with a set screw inside the nail in response to a load applied to the system by a subject, in accordance with aspects of the present disclosure.
Figure 7 is a partially sectional and exploded view of yet another exemplary embodiment of a hip fixation system with load-controlled dynamization, with the system attached to a fractured femur and including a support member structured as a side plate having a barrel portion that contains a deformable member and also including a fixation element received in the barrel portion and configured to apply a deforming force to the deformable member in cooperation with a stop member of the system, in accordance with aspects of the present disclosure.
Figure 8 is a partially sectional view of still another exemplary embodiment of a hip fixation system with load-controlled dynamization via plastic deformation of a deformable member of the system, with the system attached to a fractured femur and including a plurality of deformable spacer elements each capable of plastic deformation in response to a load applied to the system, in accordance with aspects of the present disclosure.
Figure 9 is a front view of still yet another exemplary embodiment of a hip fixation system with load-controlled dynamization via plastic deformation of a deformable member of the system, with the system attached to a fractured femur and including a support member mounted to the lateral cortex of the femur and also including a fixation element anchored in the femoral head and attached to a deformable member that is deformed by contact with a stop member of the support member, in accordance with aspects of the present disclosure.
Figure 10 is a side view of the hip fixation system of Figure 9, taken generally along line 10-10 of Figure 9 in the absence of the femur.
Figure 11 is a fragmentary front view of the hip fixation system of Figure 9, taken after deformation of the deformable member and associated axial movement of the support member and the fixation element relative to one another.

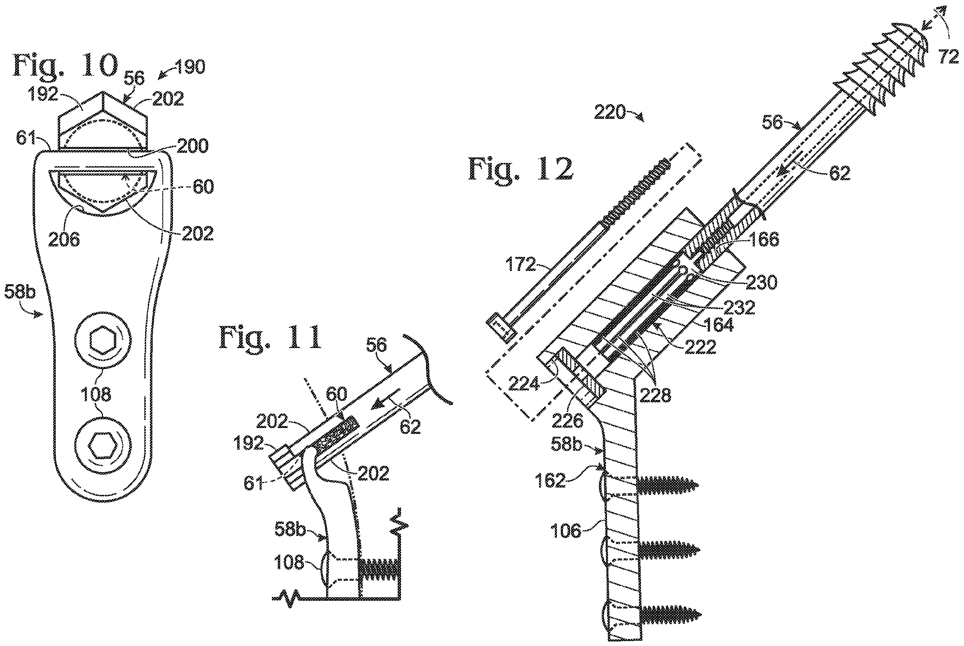

Figure 12 is a partially sectional and exploded view of an example of a hip fixation system with friction-limited dynamization, not forming part of the claimed invention, with the system including a support member in the form of a side plate having an internally tapered barrel portion, with a fixation element extending into the barrel portion and having a deformable trailing portion, and with the barrel portion providing an increasing resistance to axial travel of the side plate and the fixation element relative to one another as the barrel portion moves toward the leading end of the fixation element, in accordance with aspects of the present disclosure.
Figure 13 is a somewhat schematic sectional view of the side plate of Figure 12, taken as in Figure 12 but with the internal taper of the barrel portion exaggerated.
Figure 14 is a partially sectional and exploded view of an example of a hip fixation system having load-controlled dynamization governed by a ratchet, not forming part of the claimed invention.
Figure 15 is a partially sectional and exploded view of another example of a hip fixation system having load-controlled dynamization governed by a ratchet, not forming part of the claimed invention.
Figure 16 is a partially sectional view of a yet another example of a hip fixation system having load-controlled dynamization governed by a ratchet, not forming part of the claimed invention.

### Detailed Description

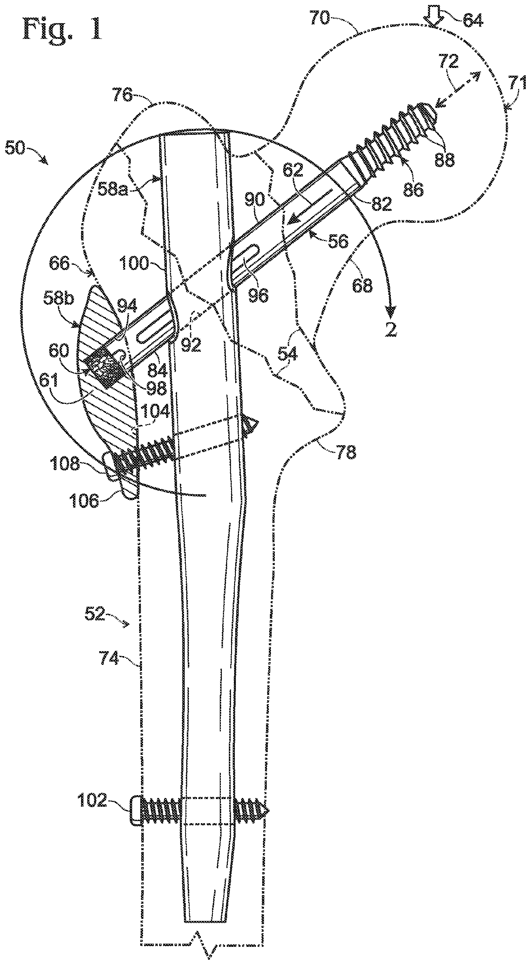

The present disclosure provides a system for hip fixation with load-controlled dynamization. According to the claimed invention, which is illustrated only in figures 1-5 and 7-11, the system comprises a fixation element, such as a screw, configured to be placed into a proximal femur of a subject, with a leading end of the fixation element anchored in a head of the proximal femur. The system also comprises a stop member configured to be connected to the lateral portion of the the proximal femur. The system further comprises a deformable member configured to be plastically deformed by compressive force exerted on at least a portion of the deformable member by the fixation element and the stop member in response to a load applied to the proximal femur by the subject, such that the fixation element and the stop member move relative to one another parallel to a long axis of the fixation element.

The load-controlled dynamization disclosed herein may have various advantages over other hip fixation systems. The ability of system components to move relative to one another in a load-controlled manner reduces the incidence of cutout of the fixation element through the femoral head, relative to a system in which system components are rigidly attached to one another. Controlling and limiting movement of system components reduces femoral shortening and establishes a limit for the amount of femoral shortening that can occur, relative to a system where system components are freely slidable relative to one another.

Further aspects of the present disclosure are described in the following sections: (I) overview of an exemplary hip fixation system, (II) methods of hip fixation, (III) composition of system components, (IV) kits, and (V) examples.

### I. Overview of an Exemplary Hip Fixation System

This section provides an overview of an exemplary hip fixation system configured for load-controlled dynamization through deformation of a deformable region of the system; see Figures 1-5.

Load-controlled dynamization may permit post-surgical compression of a femoral fracture to accommodate settling of the fracture (e.g., shortening of the femoral neck). "Load-controlled dynamization," as used herein, is any movement and/or change in position of portions of the hip fixation system relative to one another that is caused by a load applied to the hip fixation system and/or the femur, typically by a subject into which the system has been installed. The dynamization may be "distance-limited," which means that additional movement within the fixation system requires increasing force according to the degree of movement and/or change in position that the fixation system has already undergone, optionally with a maximum distance of dynamization predefined for the system. Load-controlled dynamization of the hip fixation system generally results in a corresponding load-controlled dynamization of portions of the femur relative to each other, such as dynamization of the femoral head (a medial portion of the femur) and the femoral body/shaft (a lateral portion of the femur) relative to one another.

Figure 1 shows a front view of selected aspects of a hip fixation system 50 attached to a femur 52 having at least one discontinuity, such as a fracture 54 or a cut. Fracture 54 may include an intracapsular fracture (e.g., a neck fracture) and/or an extracapsular fracture (e.g., an intertrochanteric fracture).

System 50 may include at least one fixation element 56 (e.g., one, two, three, or more fixation elements), one or more support members 58a, 58b operatively associated with the fixation element, a deformable member 60, and a stop member 61. The deformable member is capable of deformation that allows the fixation element and the stop member (and/or support member(s)) to move relative to one another, indicated by a motion arrow at 62, such that the femur is compressed at fracture 54. The deformation may occur after the system is installed in a subject, in response to a load 64 applied by the subject to the system and/or femur 52. The amount of deformation that occurs may vary with the applied load, within a load range that causes deformation. According to the invention, the deformable member undergoes plastic (irreversible) deformation, and may additionally undergo elastic (reversible) deformation, as the fixation element and the stop member/support member(s) move relative to one another. Furthermore, the resistance to further movement may increase according to the extent to which the deformable member has already been deformed, such that an increasing load is required for progressive movement of the system components relative to one another.

Fixation element 56 may change in length and/or axial position along its long axis, in response to load 64 (e.g., a downward force) applied to the fixation element via femur 52. The load may be applied by a recipient of the fixation system, also called a subject, generally using at least a portion of the subject's weight. In other words, the load may be applied post-surgery, such as when the subject stands, walks, or runs, among others. The maximum load applied by the subject to fixation element 56 may determine the magnitude of the change in length and/or axial position of the fixation element, optionally up to a load limit and/or within a load range (such as a predefined/particular load range). The change in length and/or axial position may be accompanied by motion of the femoral head and the femoral shaft relative to one another, which may apply compression to fracture 54 (or other discontinuity).

Fixation element 56 interchangeably may be termed a transverse member, a femoral head fastener, or a femoral head anchor. The fixation element may be configured to be implanted transversely to a long axis of femur 52. The fixation element extends from a lateral portion 66 (also called a body) of femur 52, through a femoral neck 68, and into a femoral head 70 (forming a medial portion 71 of the femur). The fixation element may be anchored to medial portion 71 and slidable with respect to lateral portion 66 of the femur. A long axis 72 defined by fixation element 56 (and/or a shaft thereof) may have any suitable transverse orientation with respect to the long axis of femur 52, such as forming an angle of at least about 100 degrees, less than about 150 degrees, or a combination thereof, among others. In exemplary embodiments, the fixation element is oriented at about 120 to 145 degrees with respect to the femoral long axis. Lateral portion 66 of the femur includes a shaft 74 of the femur and may include at least part of greater trochanter 76 and/or lesser trochanter 78.

Fixation element 56 may impose any suitable restrictions on intra-femoral motion. For example, the fixation element may restrict rotational motion of the femoral head relative to the femoral shaft, such as varus motion of the femoral head. Also, the fixation element may permit limited motion of the femoral head relative to the femoral shaft in a direction parallel to long axis 72.

The fixation element may have a leading portion 82 (interchangeably termed a spanning portion) disposed in femoral head 70, and a trailing portion 84 disposed at least partially in lateral portion 66 of the femur. (The leading portion enters bone before the trailing portion during installation.) One or both portions 82, 84 of the fixation element may be anchored in the femur. More particularly, one or both portions 82, 84 may include an anchoring structure 86, such as an external thread 88, that engages bone to restrict movement of the fixation element with respect to bone in both directions parallel to long axis 72. Accordingly, the fixation element may be a screw, such as a lag screw, with a thread only on leading portion 82. The anchoring structure may be formed integrally with a shaft 90 of the fixation element or may be provided by a discrete component that is movable relative to the shaft. Exemplary anchoring structures include an external thread, one or more barbs, one or more flanges (e.g., annular or helical flanges), at least one extendable pin (e.g., a talon), a pivotably deployed retainer, or any combination thereof, among others. In some embodiments, trailing portion 84 of fixation element 56 does not include an anchoring structure. The fixation element may be cannulated, with a bore extending axially through the leading portion, the trailing portion, or both (e.g., through the entire length of the fixation element.) In some embodiments, the fixation element may have a head near or at the trailing end of the fixation element (e.g., see Example 3).

Fixation element 56 may be disposed completely in the femur or may project from the femur, such as projecting from a lateral side of the femur as shown in Figure 1. Generally, only a fraction, if any, of the fixation element projects from the femur, such as less than 10% or 5% by length, among others.

The fixation element may extend into and/or through at least one support member 58a or 58b. For example, in the depicted embodiment, the fixation element extends through an opening 92 defined by inner support member 58a and into an opening 94 defined by outer support member 58b. Each opening 92, 94 may be a blind hole or a through-hole, among others. The fixation element may be slidably disposed with respect to at least one support member (and/or stop member 61) to permit motion parallel to long axis 72 as deformable member 60 deforms.

Fixation element 56 may be formed as only one piece or may be formed by an assembly of two or more discrete components/pieces that are placed into the femur as a unit. In some embodiments, the fixation element may be attachable to a compression screw via a trailing portion of the fixation element, after the fixation element has been placed into the femur. The compression screw may be turned to adjust a compression applied to the femur at a fracture 54 (e.g., see Examples 2, 4, and 5). The compression screw may extend through an opening defined by deformable member 60.

The fixation element may have any suitable shape. For example, the fixation element may have a circular cross section and/or may include a cylindrical shaft. In some embodiments, the shaft may include opposing ends and a lateral surface extending between the opposing ends. The lateral surface may include at least one non-cylindrical region. The non-cylindrical region may be a flat region, such as one or more longitudinal flats, or at least one trough 96, among others (e.g., see Example 1). Deformable member 60 may or may not be attached to the shaft, such that the fixation element and the deformable member can be placed into the femur as a unit. If attached, the deformable member may be attached to a non-cylindrical surface region of the shaft.

The fixation element may have a driver engagement structure 98 to allow the element to interface with a driver for advancement into bone. In the depicted embodiment, driver engagement structure 98 is a transverse notch formed at the trailing end of the element. In other embodiments, the driver engagement structure may include external facets or a socket (e.g., a hex socket), among others.

Each of support members 58a, 58b may be operatively associated with fixation element 56 by installation into the femur. A pair of members/components of a system that are "operatively associated" are positioned to allow direct contact with one another, or are separated by one or more intervening, implanted members/components of the system that collectively extend from one member/component of the pair to the other member/component of the pair. Each support member 58a, 58b (and/or stop member 61) may have a fixed position with respect to lateral portion 66 of the femur after installation.

Inner support member 58a may be a nail 100 (interchangeably termed an intramedullary nail) disposed at least predominantly inside the femur. Nail 100 may extend longitudinally in the femur. The nail may be used without outer support member 58b, and outer support member 58b may be used without the nail, or they may be used in combination as shown. The nail may be attached to bone with one or more fasteners 102 that extend into and/or through transverse openings of the nail. At least one fracture 54 or other discontinuity of the femur may be spanned by the nail.

The nail may have any suitable structure. Nail 100 may be linear or nonlinear (e.g., with a longitudinal bend of less than about 10 degrees). The nail may have a leading end region that is smaller in diameter than a trailing end region thereof. The nail may or may not be cannulated longitudinally.

Outer support member 58b may be disposed at least partially outside the femur, such as located on and/or attached to a lateral cortex 104 (interchangeably termed a lateral side) of the femur. The outer support member may include a mounting portion 106 provided by a plate device (which may be a buttress plate or a side plate, among others). The mounting portion may define openings to receive one or more fasteners 108, such as screws, to mount the support member on the femur (and/or to connect support members to one another). Each fastener 108 may or may not extend into nail 100. The outer support member also may or may not include a barrel portion that extends into the femur and receives a trailing portion of fixation element 56 (e.g., see Examples 2, 4, and 5).

Outer support member 58b may include or at least partially contain deformable member 60. The deformable member may or may not be discrete from the fixation element, each support member, and the stop member(s). The deformable member may or may not be attached to the fixation element, a support member, and/or the stop member. Accordingly, in some embodiments, the deformable member may be described as an insert and or may or may not be removable from opening 94 of support member 58b (or opening 92 of support member 58a). Fixation element 56 may extend into opening 94 (or opening 92) to allow the fixation element to apply deforming pressure to deformable member 60, such as to compress the element, with or without direct contact between the fixation element and the deformable member.

Deformable member 60 may have any suitable position relative to fixation element 56, a support member 58a or 58b, and stop member 61. In some embodiments, the deformable member may be operatively disposed between a shaft of the fixation element and the stop member (and/or support member). For example, the deformable member may be attached to a side surface region of the shaft between opposing ends of the shaft, and at least a portion of the deformable member may be disposed between the side surface region and a region of the stop member (e.g., see Example 1). Alternatively, the deformable member may be located between a trailing portion of the shaft and the stop member, and optionally coaxial to the shaft (and/or fixation element) (also see Examples 2 and 3). In some embodiments, the deformable member may be attached to a non-cylindrical surface region of the shaft, between opposing ends of the shaft, such as located in a trough defined by the shaft (see Example 1).

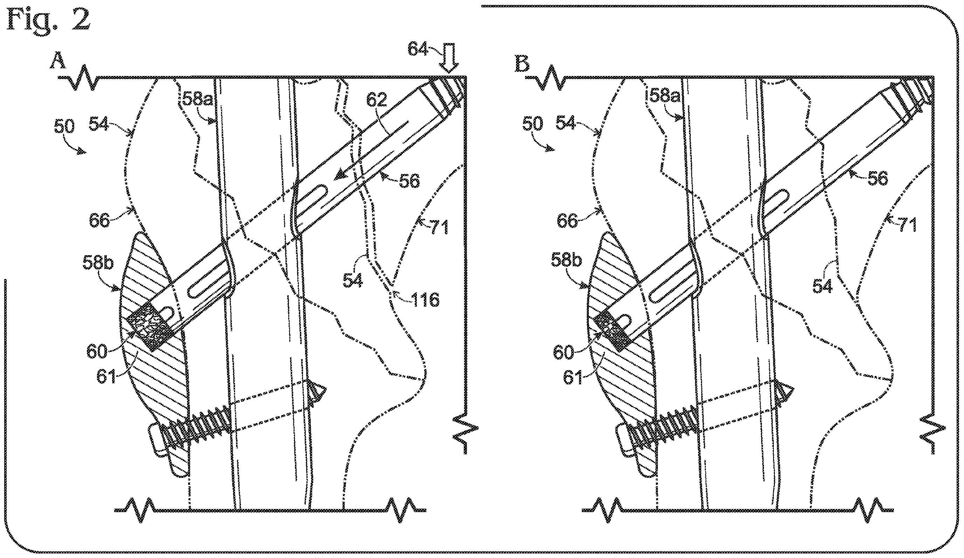

Figure 2 shows a pair of fragmentary views of fixation system 50 of Figure 1 taken before (panel A) and after (panel B) permanent compression of at least a portion of deformable member 60. The compression results in movement of fixation element 56 and outer support member 58b (including stop member 61) relative to one another, and movement of lateral and medial portions 66, 71 of the femur relative to one another (e.g., closer to one another). However, to simplify the following discussion, fixation element 56 and medial portion 71 of the femur including the femoral head are considered to be movable, while outer support member 58b, inner support member 58a, stop member 61, and lateral portion 66 of the femur, including the femoral shaft, are considered to be stationary.

Panel A of Figure 2 shows a gap 116 at fracture 54 between fracture edges of lateral and medial portions 66, 71. Accordingly, load 64 applied to the head of the femur is transmitted efficiently to fixation element 56, instead of being shared between the fixation element and abutted edges of the fracture. As a result, the load produces compression of deformable member 60 in a direction parallel to the long axis of the fixation element. The compression is accompanied by corresponding movement of fixation element 56 and outer support member 58b (including stop member 61) relative to one another, until the gap is closed (compare panels A and B) and the load is shared between the fixation element and the abutted edges of the fracture, or until the load is no longer sufficient to further compress the deformable member. Alternatively, if the fixation element is considered as stationary, outer support member 58b (including stop member 61) (and inner support member 58a) and lateral portion 66 of the femur travel inward, namely, medially and superiorly within the subject, in a direction parallel to the long axis of the fixation element. The fixation element and support member(s)/stop member may be configured to undergo any suitable maximum movement relative to one another, such as at least about 1, 2, or 5 millimeters, and/or up to about 10 millimeters, among others. In other embodiments, not forming part of the claimed invention, relative movement as described above may be permitted and controlled by friction or a ratchet, among others (e.g., see Examples 4 and 5). In some embodiments, not forming part of the claimed invention, movement of the fixation element and the support member(s)/stop member relative to one another may be configured to occur selectively in only one of two opposite axial directions parallel to the long axis of the fixation element and, optionally, without changing the orientation of the fixation element with respect to the support member(s).

In some embodiments, the fixation element may have an integral deformable member that allows the element to undergo a change in length. The length may be reduced by deforming, via plastic deformation, a selectively deformable region of the fixation element. The fixation element may undergo any suitable change in length (or axial position), such as at least about 1, 2, or 5 millimeters, and/or up to about 10 millimeters, among others. The deformable region may undergo a corresponding change in dimension.

Deformable member 60 may have any suitable structure and composition. The deformable member may have a plurality of voids that allow the member to be compressed. The deformable member may be formed of metal (such as stainless steel, titanium, etc.), a polymer, or the like. The deformable member may be compressible, which may reduce the volume occupied (e.g., reducing the volume of an imaginary envelope defined by the periphery of the deformable member). The deformable member may be compressible parallel to a first axis without substantially increasing a dimension of the deformable member transverse to the first axis. According to the invention, the deformable member is configured to be irreversibly deformable/compressible (i.e., plastically deformable). Compression of the deformable member may "crush" at least a portion of the deformable region, which is any irreversible compression. The deformable member may include a regular or random array of voids, and may have a cellular and/or porous structure.

The deformable member may be included in and/or at least partially contained by an inner support member, an outer support member, a fixation element, or a stop member, among others. Accordingly, the deformable member may be formed integrally with the support member, fixation element, or stop member, or may be inserted into, attached to, and/or formed on and/or in a support member, fixation element, or stop member.

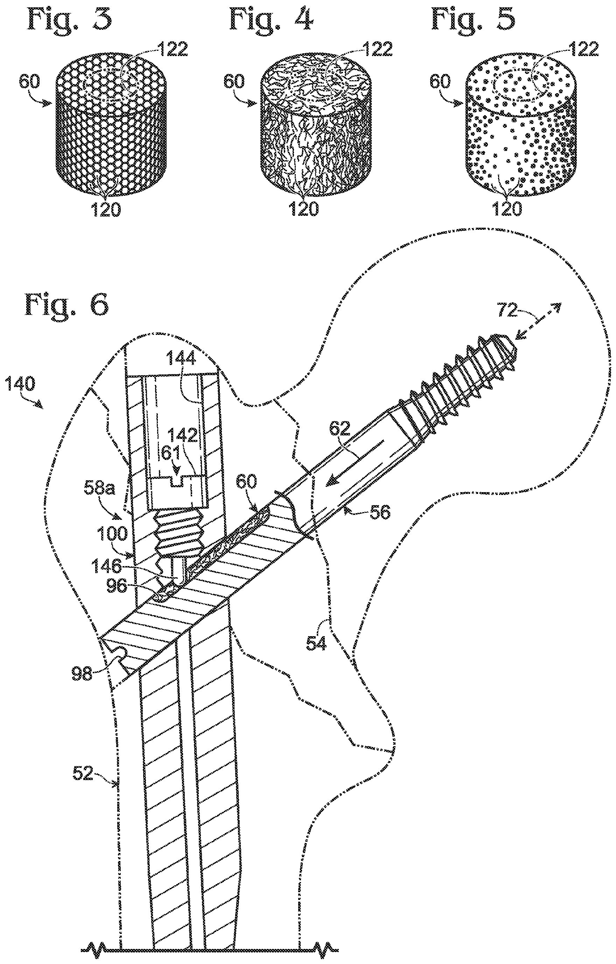

Figure 3 shows an exemplary deformable member 60 for hip fixation system 50. The deformable member may have a plurality of voids 120, which may give the deformable member a honeycomb-like structure. The deformable member may be formed by, for example, 3-D printing. The deformable member may or may not define an opening 122 (e.g., a through-hole) to receive a portion of the fixation element (such as a shaft region or a head region thereof) and/or a portion of a compression screw that attaches to the fixation element (e.g., see Examples 2 and 3).

Figure 4 shows another exemplary deformable member 60 for hip fixation system 50. The deformable member may have a porous structure, such as produced by a foam, for example, a metal foam (e.g., Duocel® foam).

Figure 5 shows still another exemplary deformable member for the hip fixation system of Figure 1. The deformable member may have a porous structure formed by a polymer or a plasma spray.

In other embodiments, the deformable member may, for example, extend transversely back and forth one or more times (e.g., to create a sinuous structure), follow a helical path, or the like.

Stop member 61 may have any suitable structure and position. The stop member may be formed integrally with a support member (e.g., support member 58b; see Figure 1 and Example 3) or may be formed separately from the support member and then attached thereto (e.g., see Examples 1 and 2). The stop member may, for example, be attached to the support member by threaded engagement. The stop member may be adjustably positionable parallel to the long axis of the fixation element (e.g., see Example 2) or parallel to the long axis of the support member (e.g., see Example 1), among others. Alternatively, the stop member may be permanently fixed with respect to the support member. The stop member may be positioned coaxially with the fixation element (e.g., see Figure 1 and Example 2), or may be adjustably offset from the central long axis of the fixation element (see Example 1).

Any of the hip fixation systems of the present disclosure also may include a compliant interface and/or a fixation element 56 that is reversibly flexible (e.g., elastic). The compliant interface (and/or flexible fixation element) may permit off-axis pivotal motion of the fixation element (and/or a region thereof) with respect to a support member (e.g., a nail or a plate) and/or with respect to the shaft of the femur, to reversibly change the orientation of at least a portion of the fixation element.

### II. Methods of Hip Fixation

This section describes exemplary methods of hip fixation using any of the fixation systems disclosed herein. The method steps described in this section may be performed in any suitable order and combination and may be combined with any other steps, and performed with any suitable devices and/or combination of device features, disclosed elsewhere herein, such as in Example 6.

A subject's femur to be fixed may be selected. The femur may have at least one discontinuity, such as at least one fracture. The at least one fracture may include an intracapsular fracture and/or an extracapsular fracture. Accordingly, the at least one fracture may include at least one fracture of the femoral body, the femoral neck, and/or the femoral head. The at least one fracture of the femoral body may include an intertrochanteric fracture and/or a subtrochanteric fracture. An intertrochanteric fracture involves the greater trochanter and/or the lesser trochanter and/or is disposed intermediate the trochanters. A subtrochanteric fracture is a fracture that is distal to the lesser trochanter.

A fixation system for fixation of the femur may be selected. The fixation system may include any combination of the elements and features described above in Section I and/or elsewhere in the present disclosure, such as in Section V. The fixation system may include at least one fixation element, one or more support members, at least one stop member, and at least one deformable member. In some embodiments, such as in the case of a femoral neck fracture, a plurality of fixation elements may be selected. A support member may provide a mounting portion for placement on the femur and/or a projecting portion for placement into the femur. The projecting portion may be a barrel portion. In some embodiments, fractures of the femoral neck may be fixed using a support member in the form of a buttress plate having a mounting portion but no projecting portion (e.g., no barrel portion), or without a support member on the femur. In some embodiments, intertrochanteric fractures may be fixed using a plate device having no projecting portion (e.g., no barrel portion), or without a plate device. The plate device may or may not be fastened to the body (e.g., the lateral cortex) of the femur with one or more fasteners (e.g., one or more fasteners that are each distinct from the fixation element).

At least one deformable member may be selected for the fixation system. Each deformable member may be provided by a set of interchangeable deformable members each having a different deformable region. The deformable regions may differ in the deformability of a deformable material that forms each deformable region, in size, in shape, or the like. Selection of a deformable member from the set may be based on at least one characteristic of a recipient of the fixation system, such as the recipient's weight, height, activity level, age, or any combination thereof, among others.

The femur may be stabilized by implanting the fixation system. The fixation element(s) may be placed in the femur, with the fixation element extending from a body to a head of the femur. If a plurality of fixation elements are placed in the femur, they may (or may not) be placed parallel to one another. A plate device may be attached to a lateral surface region of the femur and/or a nail may be placed in a medullary canal of the femur. Each fixation element may extend into (and through) the plate device and/or nail. A deformable member may be operatively disposed in the fixation system. A stop member may be operatively disposed in the fixation system.

A load may be applied to the fixation system via the femur, after the fixation system has been implanted. The load may be applied using the weight of the system recipient.

### III. Composition of System Components

Each system component may have any suitable composition. Each may be formed of any suitable biocompatible material(s) and/or bioresorbable (bioabsorbable) material(s). Illustrative biocompatible materials that may be suitable for a fixation element, a plate device, a nail, and/or a deformable member of the fixation system include (1) metal (for example, titanium or titanium alloy, cobalt-chrome alloy, stainless steel, etc.); (2) plastic (for example, ultra-high molecular weight polyethylene (UHMWPE), polymethylmethacrylate (PMMA), polytetrafluoroethylene (PTFE), polyetheretherketone (PEEK), and/or PMMA/polyhydroxyethylmethacrylate (PHEMA)); (3) bioresorbable material or polymer (for example, polymers of α-hydroxy carboxylic acids (e.g., polylactic acid (such as PLLA, PDLLA, and/or PDLA), polyglycolic acid, lactide/glycolide copolymers, etc.), polydioxanones, polycaprolactones, polytrimethylene carbonate, polyethylene oxide, poly-β-hydroxybutyrate, poly-β-hydroxypropionate, poly-δ-valerolactone, poly(hydroxyalkanoate)s of the PHB-PHV class, other bioresorbable polyesters, and/or natural polymers (such as collagen or other polypeptides, polysaccharides (e.g., starch, cellulose, and/or chitosan), any copolymers thereof, etc.)); (4) bone material or bone-like material (e.g., bone chips, calcium phosphate crystals (e.g., hydroxyapatite, carbonated apatite, etc.)); or (5) any combination thereof.

The system components may be formed of the same or different materials. For example, each may be formed of metal, each may be formed of plastic, or one or more may be formed of metal and another one or more may be formed of plastic, among others.

### IV. Kits

The hip fixation system may be provided as a system/kit including at least one fixation element, at least one plate device, at least one nail, one or more fasteners to attach the plate device/nail to bone, at least one deformable member, at least one stop member, or any combination thereof. The system/kit may provide two or more different choices for at least one of the components. For example, the system/kit may include two or more plate devices of different size and/or shape, two or more fixation elements of different size (e.g., different lengths or diameters) and/or compressibility, and/or two or more interchangeable deformable members (e.g., having a different compressibility, a different limit of compressibility, etc.).

### V. Examples

The following examples describe selected aspects and embodiments of the present disclosure including exemplary hip fixation systems with load-controlled dynamization, and methods of installing the systems to fix a femur. The components, aspects, and features of the systems, devices, and methods described in each of these examples may be combined with one another and with the systems, devices, and methods described elsewhere herein, in any suitable combination. These examples are intended for illustration and should not limit the entire scope of the present disclosure.

### Example 1. Hip Fixation System with a Nail

This example, not forming part of the claimed invention, describes another exemplary hip fixation system 140 including a nail 100 (as a support member 58a), and a fixation element 56 attached to a deformable member 60; see Figure 6.

Hip fixation system 140 may include any suitable combination of features described above, such as in Section I for system 50 (e.g., see Figures 1-5), and elsewhere in the present disclosure. However, system 140 may not utilize an outer support member 58b (compare Figures 1 and 6).

Fixation element 56 is pre-attached to a deformable member 60 that is positioned for contact with a stop member 61. The stop member is attached to nail and is adjustably positionable along the long axis of the nail by turning at least a portion of the stop member, to advance the stop member into an operative position that restricts rotation of the fixation element about its long axis and obstructs axial travel of the deformable member after the nail and fixation element have been placed into the femur. In the depicted embodiment, stop member 61 is structured as a set screw 142. In other embodiments, stop member 61 may be any advanceable and retractable member that attaches to the nail, such as by threaded engagement.

The deformable member may be located in a longitudinal trough 96 defined by a shaft of the fixation element and may extend any suitable distance along the trough, such as along only a portion or along the entire length of the trough (as shown here). The deformable member may be formed as a separate element that is mounted in trough 96 after formation or may be formed in the trough such as by application of a solidifiable material (e.g., a plasma spray or a polymerization solution) to the trough.

Screw 142 is received in an axial opening 144 defined by nail 100 and attaches to the nail via threaded engagement. The position of screw 142 along the long axis of the nail is adjustable by turning the screw. Screw 142 has a tip 146 configured be advanced into trough 96 after fixation element 56 has been placed into bone. Tip 146 prevents rotation of the fixation element about long axis 72. Also, the tip obstructs axial travel of deformable member 60. Contact between the tip and deformable member 60 limits and controls axial motion 62 of the fixation element and nail (including set screw 142) relative to one another. Deformation of deformable member 60 in response to load 64 allows limited axial motion 62 for compression of fracture 54. The stop member, such as screw 142, may or may not deform part of deformable member 60 as the screw is being advanced into trough 96 during system installation.

### Example 2. Hip Fixation System with a Side Plate Having a Barrel Portion

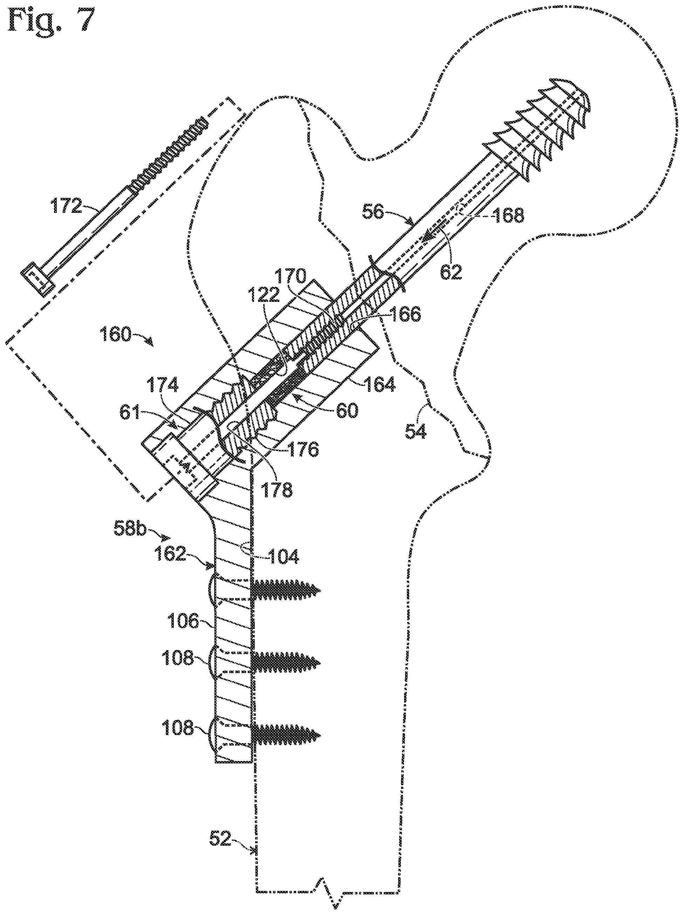

This example describes an exemplary hip fixation system 160 including a side plate 162 (as a support member 58b) having a barrel portion 164 that extends into femur 52, and also including a deformable member 60 disposed in barrel portion 164; see Figure 7.

Hip fixation system 160 may include any suitable combination of features described above, such as in Section I for system 50 (e.g., see Figures 1-5), and elsewhere in the present disclosure (e.g., Example 1). However, system 160 may not utilize an inner support member 58a (compare Figures 1, 6, and 7). System 160 may be suitable for fixation of an intertrochanteric fracture 54, among others.

Side plate 162 (also called a plate device) may include a mounting portion 106 from which barrel portion 164 projects. The mounting portion may be configured to be mounted on the proximal femur, such as mounted on a lateral surface region provided by lateral cortex 104. The mounting portion may define one or more openings to receive fasteners 108, such as bone screws, that attach the mounting portion to the femur. Mounting portion 106 and barrel portion 164 may be formed integrally with one another, or may be formed separately and assembled with one another before or during placement of the barrel portion into the femur and/or placement of the mounting portion onto the femur.

Barrel portion 164 is configured to be placed into the proximal femur from a lateral side thereof (e.g., after fixation element 56 is placed into the femur from the lateral side). The barrel portion defines a channel 166 in which the trailing end of fixation element 56 is received. The channel may be sized according the shaft diameter of the fixation element, to allow the fixation element to slide axially but to prevent off-axis movement of the fixation element. Alternatively, side plate 162 may be configured to permit changes to the orientation of the fixation element as described in U.S. Patent Application Serial No. 14/565,105, filed December 9, 2014.

Fixation element 56 may define an axial bore 168 that extends longitudinally through the fixation element. The bore allows the fixation element to be installed in the femur over a guide wire. A trailing region of the bore may include an internal thread 170 for engagement by a compression screw 172. The compression screw provides an adjustable head for the fixation element.

Barrel portion 164 also may be attached to a stop member 61. The stop member may be formed integrally with the barrel portion or may be formed separately and then attached permanently or removably to the barrel portion. In the depicted embodiment, the stop member is a removable insert 174 received in channel 166, before or after the barrel portion has been placed over the trailing end of the fixation element. Insert 174 may attach to barrel portion 164 via threaded engagement, indicated at 176, between an external thread formed by the insert and an internal thread formed inside the barrel portion by channel 166. The insert (and stop member 61 more generally) may define an axial through-hole 178 to receive the shaft of compression screw 172. The shaft of the compression screw also may extend through hole 122 of deformable member 60. A head of compression screw 172 may engage insert 174 (and stop member 61 more generally) after the compression screw has been attached to fixation element 56, to urge the fixation element toward the head of the compression screw as the compression screw is turned, to apply compression to the femur at fracture 54. The head of the compression screw may be received at least partially in a counterbore defined by through-hole 178.

A deformable member 60 may be disposed in channel 166 adjacent the trailing end of fixation element 56, and between the fixation element and stop member 61 (such as insert 174). The deformable member may be discrete and separate with respect to the stop member (such as insert 174), fixation element 56, or both. Alternatively, deformable member 60 and insert 174 (or fixation element 56) may be attached to one another before each is placed in channel 166, such that deformable member 60 and insert 174 (or fixation element 56) are installed as a unit. In any event, the insert (and stop member 61 more generally) prevents removal of deformable member 60 from the trailing end of the channel and provides a barrier for travel of the deformable member and fixation element. Furthermore, insert 174 can be manipulated (i.e., turned) to adjust the axial position of deformable member 60 along channel 166, to sandwich the deformable member between fixation element 56 and insert 174. Alternatively, system 160 may be implanted such that deformable member 60 is located between fixation element 56 and stop member 61, but is not in contact with both fixation element 56 and stop member 61 until after the system is fully implanted.

In embodiments where barrel portion 164 has an integrally formed and/or permanently attached stop member 61, deformable member 60 may be loaded into channel 166 of barrel portion 164 from the inner end thereof, before the barrel portion is placed into the femur.

### Example 3. Hip Fixation Systems for Neck Fractures

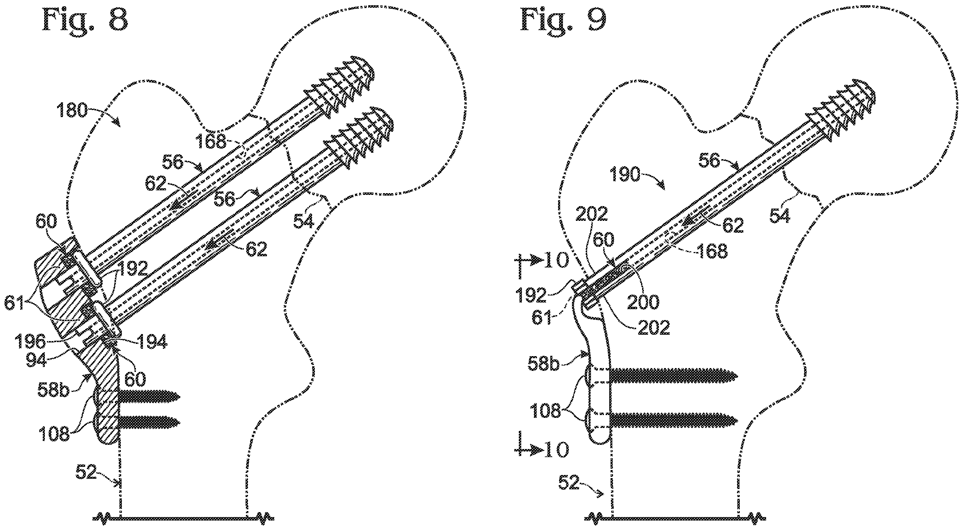

This example describes exemplary hip fixation systems 180, 190 each having a deformable member 60 and configured to fix femoral neck fractures; see Figures 8-11.

Hip fixation systems 180, 190 may include any suitable combination of features described above, such as in Section I for system 50 (e.g., see Figures 1-5), and elsewhere in the present disclosure (e.g., see Example 1). However, systems 180, 190 each may (or may not) include a plurality of fixation elements 56, which may extend parallel to one another after placement into the femur. Each fixation element 56 may or may not be cannulated. Each fixation element 56 may have a head 192 that engages a lateral surface region of the femur, to block inward travel of the fixation element into the femur, such that the fixation element applies compression to the femur at fracture 54 during installation. Systems 180, 190 may include a support member 58b in the form of a buttress plate that is mounted to a lateral cortex of the femur but that does not include a barrel portion extending into the femur (compare Figure 7 with Figures 8 and 9). Systems 180, 190 may not utilize an inner support member 58a (compare Figure 1 with Figures 8 and 9).

Support member 58b may be attached to a plurality of stop members 61 that obstruct outward travel of deformable members 60. For example, the stop members may be formed integrally with a buttress plate or may be formed separately and then attached permanently or removably to the buttress plate.

Figure 8 shows system 180 having a pair of deformable members 60 each positioned between an inner (bone-facing) surface of support member 58b and head 192 of the fixation element. Each deformable member 60 may (or may not) be disposed at least partially in a recess 194 defined in the inner surface of the support member.

Fixation element 56 may have a trailing extension 196 projecting outward from a flange forming head 192. Extension 196 extends through a deformable member 60 and is received in an opening 94 of support member 58b. Opening 94 may be contiguous with recess 194 and guides relative axial travel 62 of the fixation element and the support member relative to one another as each deformable member 60 is compressed.

Figures 9-11 show system 190 having at least one fixation element 56 containing a deformable member 60 in a longitudinal slot 200 defined by the trailing portion of the fixation element. Axial bore 168 of the fixation element may extend through deformable member 60 to the trailing end of the fixation element, such that the fixation element is cannulated. Slot 200 divides a trailing portion of the fixation element into a pair of branches 202, with deformable member 60 disposed between the branches. Support member 58b may form a stop member 61 in the form of a stop bar that is received in slot 200, to engage and compress deformable member 60. The support member defines an opening 206 adjacent the stop bar to receive one of branches 202 of fixation element 56.

### Example 4. Hip Fixation System with Friction-Limited Dynamization

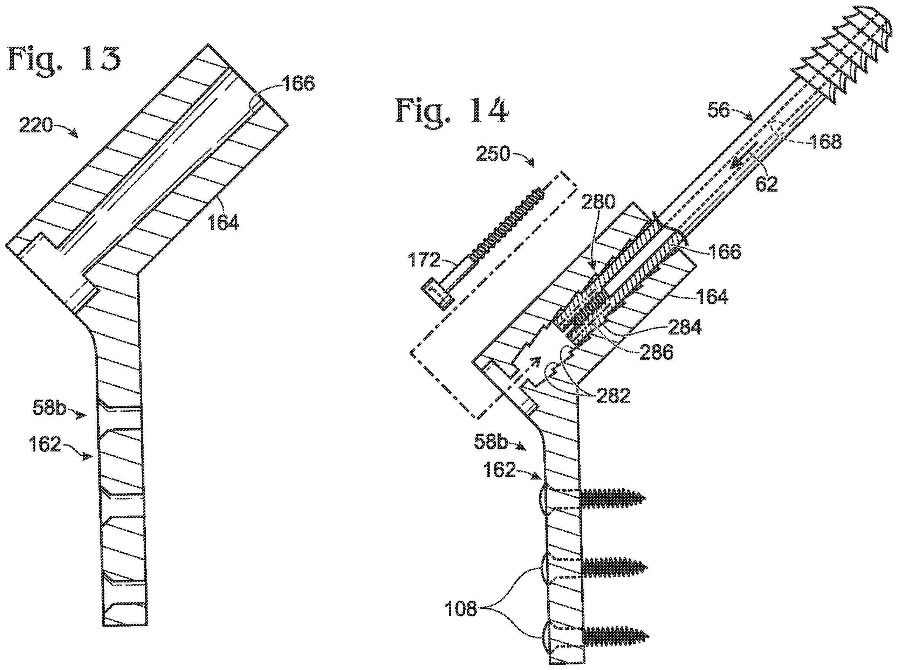

This example, not forming part of the claimed invention, describes an exemplary hip fixation system 220 including a side plate 162 having a barrel portion 164 and also including a fixation element 56 that wedges into the barrel portion; see Figures 12 and 13.

Hip fixation system 220 may include any suitable combination of features described above, such as in Section I for system 50 (e.g., see Figures 1-5), and elsewhere in the present disclosure (e.g., see Example 2). However, system 220 may have a deformable region 222 that is compressible radially (toward the central long axis of the fixation element) but not compressible deformably parallel to long axis 72.

System 220 has a barrel portion 164 with a tapered channel 166 that narrows toward the outer, trailing end of the barrel portion (and toward mounting portion 106). The outer end of the channel may widen to form a counterbore 224.

System 220 has a fixation element 56 and compression screw 172 that attach to one another as described above in Example 2, to apply compression to the femur during system installation. However, insert 174 of Example 2 is replaced by a washer 226 that seats in counterbore 224 and blocks inward travel of the head of compression screw 172 toward the femoral head as compression is applied.

Fixation element 56 may define a plurality of axial slits 228 formed in the wall of the fixation element to provide communication with a central axial bore 230. The slits separate the trailing portion into a plurality of axial branches 232 each projecting away from the leading portion of the fixation element. Each axial branch 232 is deformable radially toward the central long axis of the fixation element. Radial deformation of the axial branches produces a taper in the diameter of the trailing portion of the fixation element, toward the trailing boundary thereof. Accordingly, since the inner diameter of the barrel portion also is tapered, the inside wall of the barrel portion in combination with deformable region 222 of the fixation element provide an increasing resistance to axial motion 62 of fixation element 56 and side plate 162 relative to one another, as the trailing portion of the fixation element is wedged into the barrel portion.

Figure 13 shows barrel portion 164 with the internal taper of channel 166 more readily visible.

### Example 5. Hip Fixation Systems with a Ratchet to Govern Dynamization

This example, not forming part of the claimed invention, describes exemplary hip fixation systems 250, 260, and 270 each including a support member 58b and a fixation element 56 that collectively form a ratchet 280; see Figures 14-16. The ratchet provides an incrementally increasing resistance to axial travel 62 of fixation element 56 and the support member relative to one another.

Hip fixation systems 250, 260, and 270 each may include any suitable combination of features described above, such as in Section I for system 50 (e.g., see Figures 1-5), and elsewhere in the present disclosure (e.g., see Examples 2-4). However, system 220 may not utilize an inner support member 58a (compare Figures 1 and 14-16) and may have an elastically deformable region forming part of ratchet 280.

Figure 14 shows system 250 including a side plate 162 having a barrel portion 164. Ratchet 280 may be formed by a series of teeth 282 defined in channel 166 by an inside wall of barrel portion 164, and at least one catch 284 (interchangeably termed a detent or pawl) defined by fixation element 56 on a shaft thereof. The catch is configured to be received in the interdental spaces (the notches) formed between teeth 282. Catch 284 may or may not be formed integrally with the shaft of the fixation element. Teeth 282 may define a diameter of channel 166 that varies along the channel to produce a series of minimum diameters alternating with a series of maximum diameters. The minimum diameters may decrease toward the trailing end of the barrel portion, such that incremental motion of catch 284 toward the outer surface of side plate 162 past each tooth 282 requires increasing force. Incremental motion of the catch in the reverse direction is selectively restricted by the asymmetrical profile of each tooth. In some embodiments, fixation element 56 may define one or more axial slits 286 that facilitate radial deformation of the fixation element near catch 284, to allow the catch to move radially inward as the catch moves between interdental spaces.

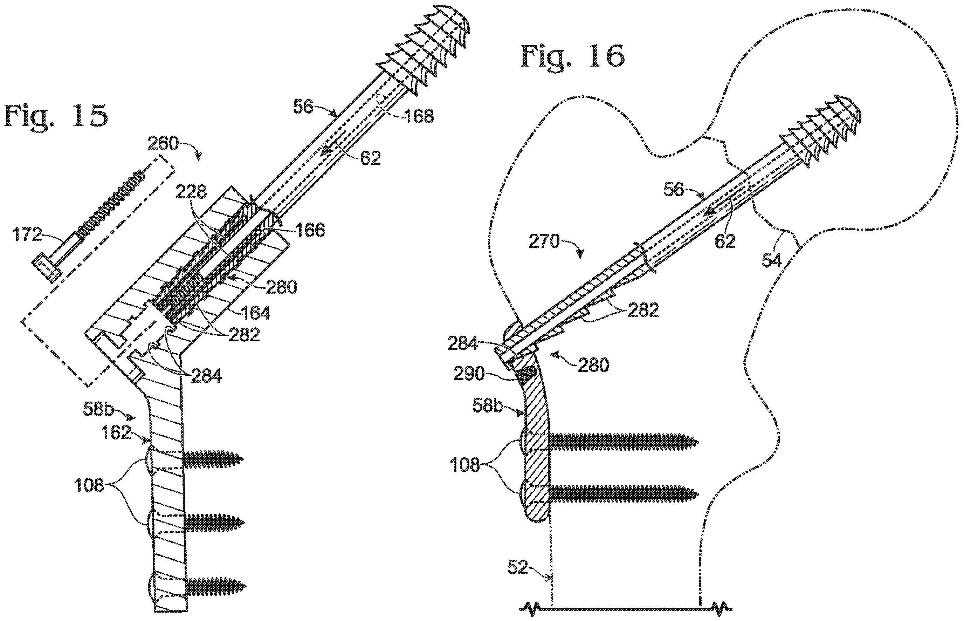

Figure 15 shows system 260, which combines features of system 250 and system 220 (also see Figures 12 and 14). Ratchet 280 of system 260 is generally similar to that of system 250 except that teeth 282 are formed on the shaft of fixation element 56 and one or more catches 284 are formed by barrel portion 164 in channel 166. Fixation element 56 has slits 228 that allow radial deformation of the trailing portion of the fixation element. Catches 284 may be formed integrally with or separately from the barrel portion. The diameter of teeth 282 may decrease toward the trailing end of fixation element 56, and/or the diameter of catches 284 may decrease toward the trailing end of channel 166, such that tooth-by-tooth incremental axial motion 62 of the fixation element and side plate relative to one another requires increasing force.

Figure 16 shows system 270 for fixation of neck fractures. The system may include one or a plurality of fixation elements 56 that each form a ratchet 280 with support member 58b, which is structured as a buttress plate. The ratchet is created by a series of teeth 282 arranged along a shaft of fixation element, and a catch formed by support member 58b. Here, catch 284 is created by an integral lip of an opening defined by the buttress plate. The lip may be movable elastically with respect to the main portion of the support member, with the aid of an elastically deformable region 290 of the support member. In other embodiments, catch 284 may be formed by a discrete component(s) with respect to the main portion of the support member.

The invention is defined and limited only by the scope of the appended claims.

## Claims

1. A system for hip fixation (50, 160, 180, 190) comprising:
a fixation element (56) configured to be placed into a proximal femur (52) of a subject such that a leading end (82) of the fixation element (56) is anchored in a head (70) of the proximal femur (52) and the fixation element (56) extends from a lateral portion (66) of the proximal femur (52) to the head (70) of the proximal femur (52);
a stop member (61) configured to be connected to the lateral portion (66) of the proximal femur (52); and
a deformable member (60) configured to be operatively disposed between the fixation element (56) and the stop member (61) and plastically deformed by compressive force exerted on at least a portion of the deformable member (60) by the fixation element (56) and the stop member (61) in response to a load (64) applied to the proximal femur (52) by the subject, such that the fixation element (56) and the stop member (61) move relative to one another parallel to a long axis of the fixation element (56).

2. The system (50, 160, 180, 190) of claim 1, wherein the fixation element (56) has a shaft (90), and wherein the deformable member (60) is attached to the shaft (90) such that the fixation element (56) and the deformable member (60) are configured to be placed into the proximal femur (52) as a unit.

3. The system (50, 160, 180, 190) of claim 2, wherein the deformable member (60) is attached to a side surface region of shaft (90).

4. The system (50, 160, 180, 190) of claim 3, wherein
(i) the side surface region of the shaft (90) defines a trough (96), and wherein the deformable member (60) is located in the trough (96); or
(ii) wherein the side surface region forms a flat region of the shaft (90), and wherein the deformable member (60) is attached to the flat region of the shaft (90).

5. The system (50, 160, 180, 190) of claim 1, further comprising a side plate (162) including a mounting portion (106) to attach the side plate (162) to a lateral cortex (104) of the proximal femur (52) and also including a barrel portion (164) configured to be placed into the femur, the barrel portion (164) defining a channel (166) to receive a trailing portion of the fixation element (56), wherein the deformable member (60) is configured to be disposed in the channel (166) between the stop member (61) and the trailing portion of the fixation element (56), optionally further comprising a compression screw (172) configured to extend through the stop member (61) and the deformable member (60) and into threaded engagement with the fixation element (56).

6. The system (50, 160, 180, 190) of claim 1, wherein the system comprises
a plurality of fixation elements (56) each configured to be placed into a same proximal femur (52) of a subject such that a leading end (82) of each fixation element (56) is anchored in a head (70) of the proximal femur (52) and the fixation element (56) extends from a lateral portion (66) of the proximal femur (52) and to the head (70) of the proximal femur (52);
a deformable member (60) operatively associated with each fixation element (56); and
a buttress plate configured to be attached to the proximal femur and including a stop member (61) for each fixation element (56).

## Patentansprüche

1. Ein System zur Hüftfixierung (50, 160, 180, 190)
umfassend:
ein Fixierungselement (56), das konfiguriert ist, um es in einem proximalen Femur (52) eines Subjekts zu platzieren, so dass ein vorderes Ende (82) des Fixierungselements (56) in einem Kopf (70) des proximalen Femurs (52) verankert ist und das Fixierungselement (56) sich von einem lateralen Abschnitt (66) des proximalen Femurs (52) zum Kopf (70) des proximalen Femurs (52) erstreckt;
ein Anschlagsteil (61), das konfiguriert ist, so dass es mit dem lateralen Abschnitt (66) des proximalen Femurs (52) verbunden werden kann; und
ein verformbares Teil (60), das konfiguriert ist, so dass es operativ zwischen dem Fixierungselement (56) und dem Anschlagsteil (61) und plastisch durch eine Druckkraft verformt wird, die auf mindestens einen Abschnitt des verformbaren Teils (60) durch das Fixierungselement (56) und das Anschlagsteil (61) ausgeübt wird, als Reaktion auf eine von dem Subjekt auf den proximalen Femur (52) ausgeübte Last (64), so dass sich das Fixierungselement (56) und das Anschlagsteil (61) relativ zueinander parallel zu einer Längsachse des Fixierungselements (56) bewegen.

2. Das System (50, 160, 180, 190) gemäß Anspruch 1, wobei
das Fixierungselement (56) einen Schaft (90) aufweist, und wobei das verformbare Teil (60) am Schaft (90) befestigt ist, so dass das Fixierungselement (56) und das verformbare Teil (60) konfiguriert sind, so dass sie als Einheit in den proximalen Femur (52) eingesetzt werden können.

3. Das System (50, 160, 180, 190) gemäß Anspruch 2, wobei das verformbare Teil (60) an einem Seitenflächenbereich des Schafts (90) befestigt ist.

4. Das System (50, 160, 180, 190) gemäß Anspruch 3, wobei
(i) der Seitenflächenbereich des Schafts (90) eine Mulde (96) definiert, und wobei das verformbare Teil (60) sich in der Mulde (96) befindet; oder
(ii) wobei der Seitenflächenbereich einen flachen Bereich des Schafts (90) bildet, und wobei das verformbare Teil (60) an dem flachen Bereich des Schafts (90) befestigt ist.

5. Das System (50, 160, 180, 190) gemäß Anspruch 1, weiterhin umfassend eine Seitenplatte (162), die einen Befestigungsabschnitt (106) zum Befestigen der Seitenplatte (162) an einer lateralen Kortex (104) des proximalen Femurs (52) beinhaltet und auch einen Zylinderabschnitt (164) beinhaltet, der konfiguriert ist, so dass er in den Femur platziert werden kann, wobei der Zylinderabschnitt (164) einen Kanal (166) zur Aufnahme eines hinteren Abschnitts des Fixierungselements (56) definiert, wobei das verformbare Teil (60) konfiguriert ist, so dass es in dem Kanal (166) zwischen dem Anschlagselement (61) und dem hinteren Abschnitt des Fixierungselements (56) angeordnet werden kann, optional weiterhin umfassend eine Druckschraube (172), die konfiguriert ist, so dass sie sich durch das Anschlagselement (61) und das verformbare Teil (60) erstreckt und in den Gewindeeingriff mit dem Befestigungselement (56).

6. Das System (50, 160, 180, 190) gemäß Anspruch 1, wobei das System eine Vielzahl von Fixierungselementen (56) umfasst, die jeweils konfiguriert sind, so dass sie in den selben proximalen Femur (52) eines Subjekts platziert werden können, so dass ein vorderes Ende (82) des Fixierungselements (56) in einem Kopf (70) des proximalen Femurs (52) verankert ist und das Fixierungselement (56) sich von einem lateralen Abschnitt (66) des proximalen Femurs (52) zum Kopf (70) des proximalen Femurs (52) erstreckt;
ein verformbares Teil (60), das operativ mit jedem Fixierungselement (56) verbunden ist; und
eine Strebeplatte, die so konfiguriert ist, dass sie am proximalen Femur befestigt werden kann und ein Anschlagsteil (61) für jedes Fixierungselement (56) beinhaltet.

## Revendications

1. Système pour fixation de hanche (50, 160, 180, 190) comprenant :
un élément de fixation (56) configuré pour être placé dans un fémur proximal (52) d'un sujet de telle sorte qu'une extrémité avant (82) de l'élément de fixation (56) est ancré dans une tête (70) du fémur proximal (52) et l'élément de fixation (56) s'étend d'une partie latérale (66) du fémur proximal (52) à la tête (70) du fémur proximal (52) ;
un élément de butée (61) configuré pour être relié à la partie latérale (66) du fémur proximal (52) ; et
un élément déformable (60) configuré pour être disposé de manière fonctionnelle entre l'élément de fixation (56) et l'élément de butée (61) et déformé de manière plastique par une force de compression exercée sur au moins une partie de l'élément déformable (60) par l'élément de fixation (56) et l'élément de butée (61) en réponse à une charge (64) appliquée au fémur proximal (52) par le sujet, de telle sorte que l'élément de fixation (56) et l'élément de butée (61) se déplacent l'un par rapport à l'autre parallèlement à un axe long de l'élément de fixation (56).

2. Système (50, 160, 180, 190) selon la revendication 1, dans lequel l'élément de fixation (56) comprend un arbre (90), et l'élément déformable (60) est fixé à l'arbre (90) de telle sorte que l'élément de fixation (56) et l'élément déformable (60) sont configurés pour être placés dans le fémur proximal (52) sous la forme d'une unité.

3. Système (50, 160, 180, 190) selon la revendication 2, dans lequel l'élément déformable (60) est fixé à une région de surface latérale de l'arbre (90).

4. Système (50, 160, 180, 190) selon la revendication 3, dans lequel
(i) la région de surface latérale de l'arbre (90) définit un creux (96), et l'élément déformable (60) étant situé dans le creux (96) ; ou
(ii) la région de surface latérale forme une région plate de l'arbre (90), et l'élément déformable (60) étant fixé à la région plate de l'arbre (90).

5. Système (50, 160, 180, 190) selon la revendication 1, comprenant en outre une plaque latérale (162) comprenant une partie de montage (106) pour fixer la plaque latérale (162) à un cortex latéral (104) du fémur proximal (52) et comprenant également une partie cylindre (164) configurée pour être placée dans le fémur, la partie cylindre (164) définissant un canal (166) pour recevoir une partie arrière de l'élément de fixation (56), l'élément déformable (60) étant configuré pour être disposé dans le canal (166) entre l'élément de butée (61) et la partie arrière de l'élément de fixation (56), facultativement comprenant en outre une vis de compression (172) configurée pour s'étendre à travers l'élément de butée (61) et l'élément déformable (60) et en engagement fileté avec l'élément de fixation (56).

6. Système (50, 160, 180, 190) selon la revendication 1, dans lequel le système comprend
une pluralité d'éléments de fixation (56) chacun configuré pour être placé dans un même fémur proximal (52) d'un sujet de telle sorte qu'une extrémité avant (82) de chaque élément de fixation (56) est ancrée dans une tête (70) du fémur proximal (52) et l'élément de fixation (56) s'étend à partir d'une partie latérale (66) du fémur proximal (52) et jusqu'à la tête (70) du fémur proximal (52) ;
un élément déformable (60) associé de manière fonctionnelle avec chaque élément de fixation (56) ; et
une plaque de soutien configurée pour être fixée au fémur proximal et comprenant un élément de butée (61) pour chaque élément de fixation (56).
